# EUROPEAN PATENT APPLICATION

(11) **EP 4 721 808 A1**
(43) Date of publication of application: **08.04.2026**
(21) Application number: 23939430.7
(22) Date of filing: 31.05.2023
(51) Int. Cl.: A61N 1/32, A61N 1/06, A61N 5/06

(54) **COMBINED DIATHERMY, ELECTROPHOROPORATION AND PHOTOSTIMULATION UNIT**

(71) Applicant: Skeyndor, S.L.U., 08223 Terrassa Barcelona (ES)
(72) Inventor: MORCILLO MUR, Jordi, 08223 Terrassa (Barcelona) (ES); MORCILLO MUR, Andrés, 08223 Terrassa (Barcelona) (ES)
(74) Representative: Zuazo Araluze, Alexander
(86) International application number: PCT/ES2023/070359
(87) International publication number: WO 2024/246375

(57) **Abstract**

The invention relates to a unit that comprises three modules the signals of which are combined in corresponding handpieces: a module for generating diathermy signals, a module for generating electrophoroporation signals, and a module for generating photostimulation signals. The three modules are electrically and electromagnetically insulated from each other. The unit further comprises a diathermy handpiece that is connected to the module for generating diathermy signals and one or more combined electrophoroporation and photostimulation handpieces that are connected to the modules for generating electrophoroporation and photostimulation signals. Each of the combined handpieces comprises a head comprising at least one photostimulation LED and electrophoroporation electrodes. The combined head comprises an application surface that is structurally divided into three parts: an inner circle (electrode), an outer annulus (electrode) and an intermediate annulus (LEDs).

## Description

### Field of the Art

The present invention relates to the technical field of facial and body aesthetics. Más particularly, the invention relates to units and treatments used in facial and body aesthetics in relation to diathermy, electrophoroporation, and photostimulation, by means of LEDs, of layers of tissues involved in said treatments.

### Background of the Invention

As is known, in the professional aesthetics field there is a wide variety of treatments that use different apparatus and products. There are also numerous technologies, and it is known that combining some of them has positive synergistic effects both technically, economically, and commercially (from the professional's point of view) and in the purely aesthetic and/or health and wellness sphere (from the perspective of the end user receiving the treatment). Among the techniques used that are relevant to the object of the present invention are diathermy, electrophoroporation, and the application of high-intensity RGB LED light.

Diathermy refers to a non-invasive treatment that increases the temperature of organs or tissues in the body by means of radiofrequency (high-frequency) electrical currents. Using an external applicator, and through the outer layers of the skin, the temperature of tissues is raised, stimulating blood flow, increasing internal temperature, and treating conditions that can affect the wellbeing of muscles, joints, ligaments, etc. Within diathermy, two types are most common: capacitive diathermy (to transfer heat to the most superficial tissues) and resistive diathermy (to act on the deepest tissues of the skin). Capacitive diathermy is normally used for softer, more vascularized tissues. Basically, two opposing electrodes are used on two different parts of the body such that the alternating polarity of said two electrodes causes the internal tissues to heat up. One of the electrodes (the handpiece or head) is smaller and is focused on the tissue to be treated (handled by the professional), while the other electrode is larger and acts as a return plate. In capacitive diathermy, the handpiece has an insulating cover on the part that comes into direct contact with the user's skin.

Electrophoroporation is a hybrid technique that combines electroporation and electrophoresis to promote the penetration of hydrophilic, low molecular weight substances through the skin. Electroporation consists of applying micro-electrical impulses to cells and tissues, causing small pores in the surface layers of the skin and in the membranes of their cells to open, allowing the passage of active ingredients into same, where the repair mechanisms are located. Electrophoresis increases the penetration of electrically charged active ingredients through the hair follicles and sweat glands, as a result of the application of a continuous microcurrent. This achieves non-invasive, faster, safer, and highly effective penetration of substances without the need for needles or surgery. This technology achieves penetration several times greater than that achieved by means of topical application. It is used in aesthetics for conditions such as cellulite, stretch marks, sagging skin, wrinkles and expression lines, lack of skin hydration, pigmentation problems, redness, and rosacea, among others.

High-intensity RGB LEDs are a relatively new technique. However, it is a highly versatile and very useful technique in the field of aesthetics and dermatology due to its numerous benefits, which are very effective and minimally invasive. It is a system of LED lightemitting diodes with a reduced spectrum when current passes through their terminals. LED light therapies use up to seven lights corresponding to the visible spectrum, defined by physical parameters such as dose intensity, flux, and wavelength. The different colors of visible light have different effects on cells and tissues, depending on their capacity to penetrate the skin and photonic energy.

The combination of the three techniques mentioned above and the relevant cosmetic products achieves a series of beneficial effects on a variety of aesthetic problems, such as excess sebum and acne, loss of light, excessive pigmentation, loss of tone and tissue volume, etc., which currently must be treated in separate sessions and/or with different units intended for each of these techniques. It would therefore be advantageous to have a single unit for all of said techniques, with the resulting advantages of space savings and functional versatility for professionals in the field, as well as convenience for the client or user when receiving the combined treatment. The combination is not only achieved at the level of these three techniques but also in terms of its application to the face and body, all in a single unit. Furthermore, combining at least two of the techniques in the same treatment session leads to advantages relating to lower energy consumption and a reduction in waste generated, for example, by consumables or packaging, which would otherwise multiply in the case of individual, successive sessions.

### Description of the Invention

As set forth above, a combined diathermy, electrophoroporation and photostimulation unit is provided. The unit comprises three independent modules but which signals are combined in corresponding handpieces (heads): a module for generating diathermy signals, a module for generating electrophoroporation signals, and a module for generating photostimulation signals. The three modules, preferably contained in the same housing, are electrically and electromagnetically insulated from each other. The unit further comprises a diathermy handpiece (there may be several of them) that is connected to the module for generating diathermy signals and one or more combined electrophoroporation and photostimulation handpieces that are connected to the modules for generating electrophoroporation and photostimulation signals. Each of the combined handpieces comprises a head comprising at least one photostimulation LED (preferably several) and electrophoroporation electrodes (preferably two in each handpiece).

According to a feature of the invention, the combined photostimulation and electrophoroporation head comprises an application surface that is circular and structurally divided into three parts: an inner circle, an outer annulus, and an intermediate annulus, between the inner circle and the outer annulus. The inner circle and the outer annulus form the two electrophoroporation electrodes. The intermediate annulus of the application surface is made of or comprises a material that allows the passage of light (translucent or even transparent) from one or more LED diodes mounted internally for this purpose.

According to another feature of the invention, the LED or LEDs comprised in each head of the combined handpieces are preferably red, blue, and green light LEDs.

According to another feature of the invention, directly related to the feature set forth above, a combined head may comprise three red LEDs, three green LEDs, and three blue LEDs. These nine LEDs will be arranged in a repetitive pattern of three different colors, for example, red LED, green LED, and blue LED, distributed uniformly throughout the intermediate annulus.

According to an additional feature of the invention, the wavelength of the red LEDs is between 620 and 750 nm, the wavelength of the green LEDs is between 495 and 570 nm, and the wavelength of the blue LEDs is between 450 and 495 nm.

Likewise, according to another feature related to the mentioned LEDs, the light output of the red LEDs is substantially 84 lumens, the light output of the green LEDs is substantially 120 lumens, and the light output of the blue LEDs is substantially 72 lumens. The wavelengths and outputs set forth herein are optimal when combined with the products or substances containing suitable active ingredients. This does not mean that beneficial results cannot also be obtained with other different outputs and wavelengths, so these values merely exemplify a preferred embodiment.

According to a further feature of the invention, the unit comprises preferably two combined electrophoroporation and photostimulation handpieces. One of the units, the one with the smallest diameter, is intended for being applied on the user's/patient's face, and the other unit, the one with the largest diameter, is intended for areas of the body. In a preferred embodiment, the selected diameters are substantially 3 cm and 6 cm.

According to an additional feature of the invention, the output voltage between the electrophoroporation electrodes in the two combined handpieces mentioned in the preceding paragraph is 60 and 30 volts, respectively, for the one with the largest diameter and the one with the smallest diameter. In relation to this, another feature of the invention is that the signal applied to the electrophoroporation electrodes comprises a frequency of 2.1 kHz modulated at 2 Hz or at 4 Hz.

Another further feature of the invention lies in the fact that the application surface of the heads of the diathermy handpieces comprises a thermoplastic, biocompatible insulating cover certified for medical-aesthetic use. In a preferred embodiment, this cover comprises or is made of polyamide.

According to another feature of the invention, the frequency band of the applied diathermy signal comprises 448 kHz.

According to an additional feature of the present invention, the diathermy head of the diathermy handpiece is interchangeable, i.e., it is a detachable or removable piece that can be replaced with other similar pieces with some specific characteristic that is different. In relation to this, another feature of the invention consists of the unit comprising three of these interchangeable heads and these heads have three different circular application surfaces. In the preferred embodiment, the diameters of these surfaces are 20 mm, 40 mm, and 60 mm.

According to another feature of the invention, the module for generating diathermy signals is adapted for specifically generating capacitive diathermy signals.

Another further feature of the invention relates to the unit comprising a control interface. This control interface includes, in turn, visualization means for displaying or visualizing the applied treatments and the different options to be selected in each treatment. The interface may also include touch buttons (for example, in the visualization means per se) and/or physical buttons that can be actuated to generate the specific signals of the heads and determine, for example, the type of application, whether sequential, simultaneous, or pre-set.

According to another feature of the invention, the unit may include a support for the handpieces and a return plate that is typical in diathermy treatments.

According to another feature of the invention, the modules for generating diathermy signals, electrophoroporation signals, and photostimulation signals and the interface mentioned above are included (all or some of them) in a single housing or physical casing.

A further feature of the invention relates to the unit comprising a support with wheels, and said support can be detached from the unit (from the housing) to leave it fixed and stable in one location.

According to another feature of the invention, the unit comprises an electronic component housing and another housing herein referred to as the furniture housing. The two housings are mounted together when the unit is operating but can be separated or detached from one another. The electronic component housing comprises the module for generating diathermy signals, the module for generating electrophoroporation signals, the module for generating photostimulation signals, and the interface, or at least one of these components. The furniture housing has internal space for storing the handpieces, the return plate, and any other accessory or cable; furthermore, said space can be accessed through the rear part.

According to another feature, the electronic component housing can be mounted or stacked firmly on the furniture housing and the unit; in that case, it may also comprise a wheeled support that can be detached from the furniture housing, similarly to the case of the single housing.

According to an additional feature of the invention, either the single housing or the electronic component housing comprises a handle. The single housing or the furniture housing also may comprise a door.

According to another feature of the invention, the translucent material of the intermediate annulus (for allowing the passage of light from the LEDs) is or comprises PMMA.

Finally, according to another additional feature of the invention, the conductive material of the electrophoroporation electrodes is or comprises steel.

### Brief Description of the Drawings

In order to complement the present description and facilitate understanding of the features of the invention, the following figures are attached, depicting the following in an illustrative and non-limiting manner:
Figure 1 is a perspective view of an embodiment of a combined diathermy, electrophoroporation and photostimulation unit according to the present invention.
Figure 2 is a perspective view of an embodiment of a combined electrophoroporation and photostimulation handpiece according to the present invention.
Figure 3 is a perspective view of an embodiment of another one of the combined electrophoroporation and photostimulation handpieces included in the unit of the present invention.
Figure 4 is a detail of the head of the combined handpieces shown in Figures 2 and 3.
Figure 5 is a perspective view of an embodiment of a capacitive diathermy handpiece of the unit of the present invention.
Figure 6 is a perspective view of a return plate for the electrophoroporation handpieces of the present invention.
Figure 7 is a perspective view of another embodiment of the combined diathermy, electrophoroporation and photostimulation unit according to the present invention.

### Detailed Description of the Invention

A detailed description of the combined unit of the present invention in reference to the attached drawings is provided below.

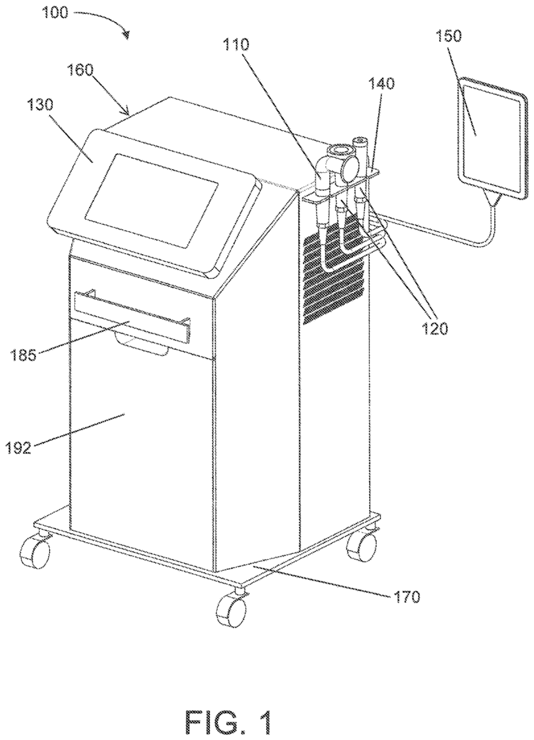

Figure 1 shows an embodiment of the combined electrophoroporation, diathermy and photostimulation unit (100) of the invention. Said figure shows a housing (160) which, in a preferred embodiment, contains the three modules that generate signals corresponding to the three mentioned treatments. Preferably, the housing (160) also contains or supports an interface (130) and the connections for the diathermy handpiece (110) and electrophoroporation/photostimulation handpiece (120) as well as for a return plate (150) required for diathermy treatment. These handpieces (110, 120) and, in another embodiment, the return plate (150), can be placed in a support (140) arranged for that purpose. In the illustrated embodiment, this support (140) comprises a shelf fixed in cantilever fashion to the housing (160) and said shelf includes recesses suitable for supporting the different components (any other type of support (140) could be used).

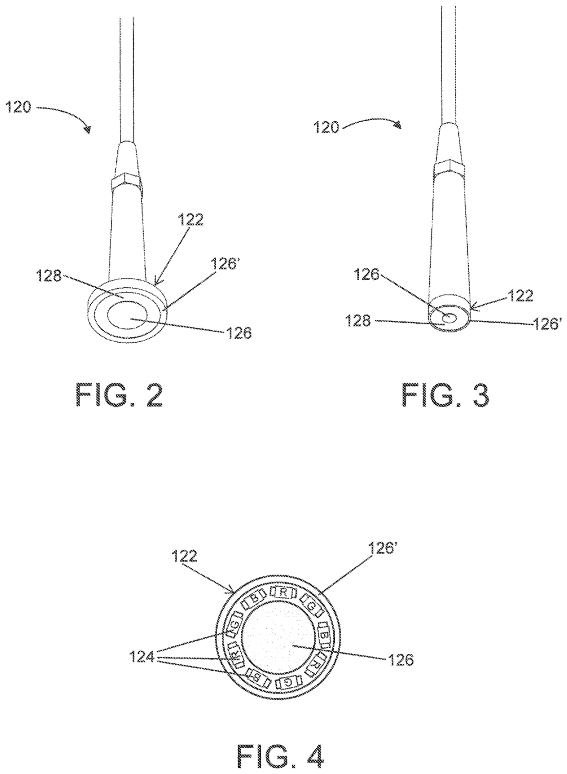

Figure 2 shows one of the combined electrophoroporation and photostimulation handpieces (120). Said figure shows the gripping part of the transducer (the handpiece *(120) per se)* and the application part, herein referred to as head (122), to distinguish between both. The head (122), intended for being in contact with the client's or patient's skin, comprises on its application surface two electrophoroporation emitting or return electrodes or poles (126, 126') and an intermediate annulus (128) for applying photostimulation by means of LEDs. Figure 3 shows another handpiece (120) with its corresponding head (122) which differs from the one shown in Figure 2 only by the dimensions of the application part, so the same reference numbers are used as they designate identically constituted elements or parts. As was just mentioned, the difference lies in the fact that the application surface of the head (122) of Figure 2 has a diameter of 3 cm (for facial application) whereas the application surface of the head (122) of Figure 3 has a diameter of 6 cm, for body application. These measurements have been found to be the maximum allowable which allow for a correct adaptation of the head to the corresponding anatomical areas of the face and body, while at the same time maintaining functionality in terms of the application of currents between the two electrodes (126, 126') and of photostimulation with the annulus (128). If diameters larger than those established herein are used, there is no good adaptation to the different irregularities of the human body and, in many cases, the different parts of the head (122) do not contact and do not transmit the signals to the target tissues. Furthermore, this unwanted separation between the application surface of the head (122) and the patient's body causes of risk of generating electric arcs between the electrodes (126, 126') and the skin which, no matter how small they may be, can be very uncomfortable, and can even cause second-degree burns to the skin.

The invention allows the combination of electrophoroporation electrodes (126, 126') and photostimulation LEDs (124) in the same handpiece (120). These, combined with suitable active ingredients, provide the advantages described herein. Although it shows beneficial results without the application of active ingredients, photostimulation by high-intensity LEDs (124) is noticeably enhanced with the aid of photostimulators. Moreover, electrophoroporation electrodes (126, 126') apply micro-electrical impulses (electroporation) that open up small pores in the surface layers of the skin and in the membranes of their cells; this temporary opening of those micropores facilitates the action of the applied active ingredients. Electrophoresis (which is combined with electroporation in electrodes (126, 126') to give rise to electrophoroporation) increases the penetration of electrically charged active ingredients through the hair follicles and sweat glands, as a result of the application of a continuous microcurrent. In summary, this head (122) will create the conditions necessary for the massive and simultaneous infusion of the active ingredients: with net charge (hydroelectrophoresis), without a charge (phoroporation), and the photostimulators (LED light), contained in the associated cosmetics.

The preferred working frequency for the electrodes (126, 126') is 2.1 KHz modulated at either 2 Hz or at 4 Hz; likewise, their output voltage is 30 and 60 volts. Current flows from one electrode (126, 126') to the other (126', 126) and has a more superficial effect compared to diathermy. The action of this application is even further enhanced in the central steel piece (negative) (126), which brings about a higher efficacy of the active ingredient. The level of current sensation is determined by unit (100), with the interface (130), depending on the intensity used and treatment duration.

The intermediate annulus (128) is made of a translucent material (in another embodiment it could also be transparent), preferably PMMA, and the electrodes (126, 126') are conductive, preferably made of steel; other materials could be used provided that they provide the characteristic of translucence or transparence for the intermediate annulus (128) and conduction for the electrodes (126, 126'). Figure 4 depicts a head (122) of a combined handpiece (120) without the translucent intermediate annulus (128) to illustratively show a preferred arrangement of the LEDs (124) inside the head (122). In other words, the LEDs (124) are fixed inside the head (122) such that their light passes unobstructed through the intermediate annulus (128) until reaching the user's skin. The characteristic of these LEDs (124) is that they are high-intensity LEDs and a preferred arrangement consists of combining red, green, and blue light LEDs (124); namely, the inventors, have observed that intensities of substantially 84 lumens for the red LEDs, 120 lumens for the green LEDs, and 120 lumens for the blue LEDs, shows good results in relation to the following characteristics.

Red light enhances the generation of collagen and elastin, as it reaches the fibroblasts located in the dermis. This provides an anti-aging effect, reducing the appearance of expression lines, wrinkles, and other signs of aging. Green light evens out facial tone, brightens dull skin, and softens blemishes and other skin imperfections. It is particularly interesting for treating hyperpigmentation, as it acts on the melanocytes located in the basal layer of the epidermis. Finally, blue light reduces the appearance of imperfections and combats the microorganism involved in the appearance of acne (*Cutibacterium acnes*); it also softens and reduces skin inflammation.

Said Figure 4 also shows a preferred arrangement of the LEDs (124), in a total number of 9, with three red, three green, and three blue LEDs in the following circumferential cyclical pattern: R G B R G B R G B. Other arrangements and numbers of LEDs are also possible, as is clear from the claims, depending on whether more or less emphasis is to be placed on each of the beneficial effects listed above for the red, green, and blue lights.

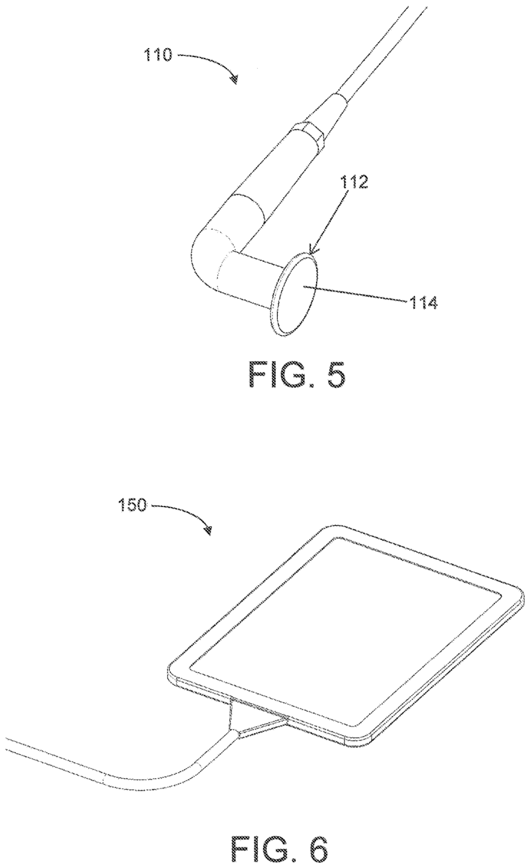

Figure 5 shows a diathermy handpiece (110) comprising a head (112) and a cover (114). This unit (100) therefore combines high-frequency monopolar capacitive radiofrequency at 448 kHz (diathermy through the head (112)) with the efficacy of the low and medium frequency currents (mentioned above for electrophoroporation) and photostimulation by LEDs in the heads (122).

Specifically, as is known, diathermy technology works with a high-frequency, low-voltage, and high-intensity electric current that generates heat. This accelerates the processes of cell regeneration and metabolism in tissues, favors the burning of fats, and the formation of collagen, and it further acts as a cell biomodulator. The cover (114) comprises a biocompatible insulating material certified for medical-aesthetic use as it must be in contact with the patient's skin; said material is also preferably thermoplastic and more preferably is or comprises polyamide. The head (112) essentially comprises the capacitive monopolar electrode for diathermy at 448 kHz and is interchangeable between three diameters of 20 mm, 40 mm, and 60 mm, respectively.

Figure 6 shows a return plate (150) for the diathermy handpiece (110). As is known, the effect of this method (diathermy) is caused by the circuit created between the head (112) and the central part (rectangular in Figure 6) of the return plate (150) which is made of a conductive material, preferably steel in this case. As is also normal in these methods, it is advisable for all or the largest possible part of the surface of said rectangular part to be in contact with the patient's skin.

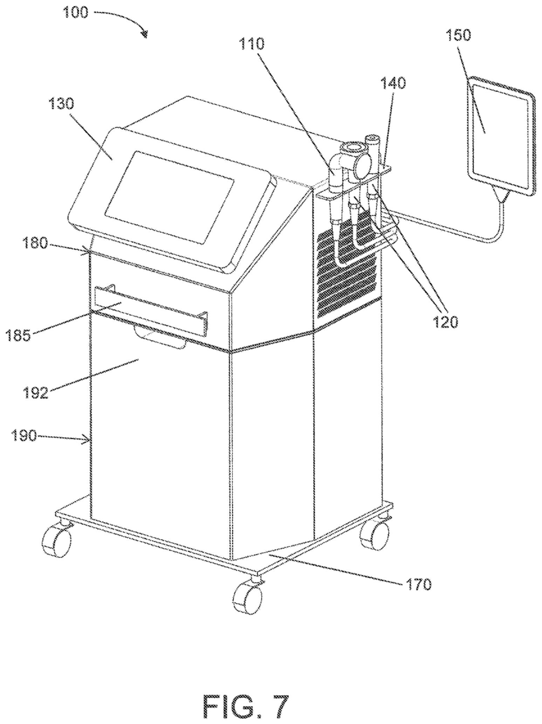

Figure 7 shows another embodiment of the unit (100) which, in this case, is made up of two housings (180, 190) which, when assembled, make up the whole of the unit (100). The housing (180) is intended for containing all the electronic components of the unit (100) and can be detached or separated from the furniture housing (190). This helps moving or transporting the electronic parts of the unit (100) requiring repair or error verification tasks, for example. In a preferred embodiment, these electronic parts will be the modules for generating diathermy, electrophoroporation, and photostimulation signals, and the interface (130). Furthermore, the housing (190) is envisaged to be accessible from the rear part to store all the handpieces (110, 120), the return plate (150), as well as cables, interchangeable diathermy heads (112), and other tools or accessories required within the professional's reach when applying the treatment. Both Figure 1 and Figure 7 further illustrate a handle (185), both in the case of the single housing (160) but especially in the case of the two housings (180, 190), useful for handling or moving the unit (100). When the housing (180) is to be separated from the furniture housing (190) for electrical/electronic maintenance or repair tasks, it will be advantageous to use said handle (185) for pulling out that part of the unit (100) taking into account the weight of the electronic components with transformers and other elements of that type. Additionally, there can be a door (192) (in the illustrated case, a hinged door, although this is just one example of the different variants obvious to the skilled person) to access the inside of housing (190) (or of housing (160) in the case of a single housing) from the front. The attachment between both housings (180, 190) is illustrated by means of the double line passing below the handle (185) and right above the door (192).

The unit (100) also comprises a wheeled support (170) to be able to readily transport the apparatus. Fixing means are provided between the unit (100) and said support (170) so as to ensure that it is moved safely. Moreover, the wheeled support (170) may be detached from housing (160) or, where appropriate, from housings (180, 190), in the event that it is to be left fixed in one position.

In one embodiment, the unit (100) comprises an infrared thermometer to be able to monitor at all times the intensity of the signals applied to the patient and to keep them within functional operating ranges and outside values that are too uncomfortable or even harmful to the user.

## Claims

1. A combined diathermy, electrophoroporation and photostimulation unit (100) comprising:
- a module for generating diathermy signals, a module for generating electrophoroporation signals and a module for generating photostimulation signals electrically and electromagnetically insulated from each other,
- at least one diathermy handpiece (110) connected to the module for generating diathermy signals and at least one combined electrophoroporation and photostimulation handpiece (120) connected to the modules for generating electrophoroporation and photostimulation signals, said combined handpiece (120) comprising a combined head (122) comprising at least one photostimulation LED (124) and electrophoroporation electrodes (126, 126').

2. The unit (100) according to claim 1, wherein the combined head (122) of the at least one combined handpiece (120) comprises a circular application surface comprising, in turn, an inner circle (126) and an outer annulus (126') that form the two electrophoroporation electrodes (126, 126'), and an intermediate annulus (128), between said inner circle (126) and said outer annulus (126'), comprising translucent material suitable for allowing the passage of light from the at least one photostimulation LED (124) arranged inside the head (122).

3. The unit (100) according to any of the preceding claims, wherein the at least one LED (124) comprises one or more red and/or blue and/or green LED(s).

4. The unit (100) according to the preceding claim, wherein the at least one LED (124) comprises three red LEDs, three green LEDs, and three blue LEDs arranged in an alternating pattern of three different colors, repeated three times, along the intermediate annulus (128).

5. The unit (100) according to any of claims 3 and 4, wherein the wavelength of the red LEDs, if there are any, is comprised between 620 and 750 nm, and/or the wavelength of the green LEDs, if there are any, is comprised between 495 and 570 nm, and/or the wavelength of the blue LEDs, if there are any, is comprised between 450 and 495 nm.

6. The unit (100) according to any of claims 3 to 5, wherein the light output of the red LEDs is substantially 84 lumens, and/or the light output of the green LEDs is substantially 120 lumens, and/or the light output of the blue LEDs is substantially 72 lumens.

7. The unit (100) according to any of the preceding claims, comprising two combined handpieces (120) with respective circular application surfaces of different diameters for facial and body application, respectively.

8. The unit (100) according to the preceding claim, wherein the smallest diameter of the two combined handpieces (120) has substantially a value of 3 cm and the largest diameter has substantially a value of 6 cm.

9. The unit (100) according to any of claims 7 to 8, wherein the output voltages between the electrophoroporation electrodes (126, 126') of the two combined handpieces (120) of the largest and smallest diameters are substantially 60 and 30 volts, respectively.

10. The unit (100) according to any of the preceding claims, wherein the signal applied to the electrophoroporation electrodes (126, 126') comprises a frequency of 2.1 kHz modulated at 2 Hz or at 4 Hz.

11. The unit (100) according to any of the preceding claims, wherein the application surface of the head (112) of the at least one diathermy handpiece (110) comprises a thermoplastic, biocompatible insulating cover (114) certified for medical-aesthetic use.

12. The unit (100) according to the preceding claim, wherein the cover (114) of the application surface of the diathermy head (112) at least partially comprises polyamide.

13. The unit (100) according to any of the preceding claims, wherein the frequency band of the applied diathermy signal comprises 448 kHz.

14. The unit (100) according to any of the preceding claims, wherein the diathermy head (112) of the at least one diathermy handpiece (110) is interchangeable.

15. The unit (100) according to claim 14, comprising three interchangeable diathermy heads (112) with circular application surfaces having substantially a diameter of 20 mm, 40 mm, and 60 mm, respectively.

16. The unit (100) according to any of the preceding claims, wherein the module for generating diathermy signals is adapted for generating capacitive diathermy signals.

17. The unit (100) according to any of the preceding claims, comprising a control interface (130) comprising, in turn, visualization means and touch and/or physical buttons for the sequential and/or simultaneous and/or pre-set visualization, generation, and application of the signals activating the heads (112, 122) of the handpieces (110, 120).

18. The unit (100) according to any of the preceding claims, comprising a support (140) for at least one of the handpieces (110, 120) and/or a diathermy return plate (150).

19. The unit (100) according to any of the preceding claims, wherein the module for generating diathermy signals and/or the module for generating electrophoroporation signals and/or the module for generating photostimulation signals and/or the interface (130) are comprised in a single housing (160).

20. The unit (100) according to any of claims 1 to 18, comprising an electronic component housing (180) and a furniture housing (190), wherein:
- both housings (180, 190) can be separated from one another, and
- the electronic component housing (180) comprises the module for generating diathermy signals and/or the module for generating electrophoroporation signals and/or the module for generating photostimulation signals and/or the interface (130), and the housing (190) is accessible through the rear part for storing the handpieces (110, 120), the return plate (150), and any other accessory or cable that can be used with the unit (100).

21. The unit (100) according to claim 20, wherein housing (180) can be mounted on housing (190).

22. The unit (100) according to any of claims 19 to 21, wherein housing (160) or housing (190) has an access door (192).

23. The unit (100) according to any of claims 19 to 22, comprising a wheeled support (170) that can be detached from housing (160) or from housing (190).

24. The unit (100) according to any of claims 19 to 23, wherein the single housing (160) or the electronic component housing (180) comprises a handle (185).

25. The unit (100) according to any of claims 2 to 24, wherein the material of the intermediate annulus (128) comprises PMMA.

26. The unit (100) according to any of the preceding claims, wherein the material of the electrophoroporation electrodes (126, 126') comprises steel.
